# EUROPEAN PATENT APPLICATION

(11) **EP 4 714 361 A1**
(43) Date of publication of application: **25.03.2026**
(21) Application number: 24815720.8
(22) Date of filing: 30.04.2024
(51) Int. Cl.: A61B 8/00, A61B 8/06, A61B 8/08

(54) **APPARATUS AND METHOD FOR PROCESSING ULTRASOUND IMAGES**

(30) Priority: 02.06.2023 KR 20230071601
(71) Applicant: Alpinion Medical Systems Co., Ltd., Hwaseong-si, Gyeonggi-do 18365 (KR)
(72) Inventor: PARK, Da Jeong, Anyang-si Gyeonggi-do 14120 (KR); CHANG, Sun Yeob, Seoul 03477 (KR); LEE, Seung Hwan, Suwon-si Gyeonggi-do 16693 (KR)
(74) Representative: Sander, Rolf
(86) International application number: PCT/KR2024/005829
(87) International publication number: WO 2024/248341

(57) **Abstract**

Disclosed are an apparatus and method for processing ultrasound images. The apparatus and method for processing ultrasound images, according to an embodiment, can dynamically and stereoscopically express a blood flow image through stereoscopic image processing by expressing the static flow of blood flow in the blood flow image on the basis of static information of blood flow data, which is independent of time changes, and simultaneously by expressing the dynamic flow of blood flow on the basis of dynamic information of the blood flow data, which changes over time.

## Description

### [Technical Field]

The present invention relates to ultrasonic technology, and more particularly, to ultrasound image processing technology.

### [Background Art]

An ultrasound apparatus transmits an ultrasound signal toward a specific tissue of a subject and obtains an ultrasound tomographic image of soft tissue, or an ultrasound image of blood flow, by using information of an ultrasound signal reflected from an internal tissue. Such an ultrasound apparatus has advantages of being compact, inexpensive, and capable of real-time display. Also, because the ultrasound apparatus does not involve exposure to radiation such as X-rays, it has high safety, and is therefore widely used along with other imaging diagnostic apparatuses such as X-ray diagnostic apparatuses, computerized tomography (CT) scanners, magnetic resonance imaging (MRI) apparatuses, and nuclear medicine diagnostic apparatuses.

### [Disclosure]

### [Technical problem]

According to an embodiment, an apparatus and method for processing ultrasound images are provided, which can represent a planar blood flow image in a stereoscopic and dynamic manner.

### [Technical Solution]

A method of processing ultrasound images, according to an embodiment, includes: acquiring ultrasound data of a blood flow (hereinafter referred to as "blood flow data") from a subject including a region of interest; calculating static information of blood flow, which is independent of time changes, and dynamic information of blood flow, which changes over time, by analyzing the blood flow data; performing stereoscopic image processing such that a static flow of blood flow using the calculated static information and a dynamic flow of blood flow using the calculated dynamic information are expressed; and outputting a stereoscopically processed blood flow image, wherein the dynamic information includes an instantaneous variation amount of blood flow.

The method may further include expressing the dynamic information, wherein the expressing of dynamic information may include, for blood flow data at a predetermined time, when the instantaneous variation amount of blood flow as the dynamic information has a positive (+) value, expressing the instantaneous variation amount as increasing, and, when the instantaneous variation amount of blood flow as the dynamic information has a negative (-) value, expressing the instantaneous variation amount as decreasing.

The performing of stereoscopic image processing may include increasing or decreasing an intensity of a light source, as a light source effect, according to a change in blood flow intensity over time, on the basis of the instantaneous variation amount of blood flow which is the dynamic information, when the instantaneous variation amount increases in a positive (+) direction, it may be determined that the blood flow intensity has increased and the intensity of the light source may be increased, and when the instantaneous variation amount increases in a negative (-) direction, it may be determined that the blood flow intensity has decreased, and the intensity of the light source may be decreased.

The performing of stereoscopic image processing may include adjusting a degree of reflection of a light source and the subject, as a light source effect, according to a change in an amount of blood flow over time, on the basis of the instantaneous variation amount of blood flow which is the dynamic information, when the instantaneous variation amount increases in a positive (+) direction, it may be determined that the amount of blood flow has increased and the degree of reflection of the light source and the subject may be increased, and when the instantaneous variation amount increases in a negative (-) direction, it may be determined that the amount of blood flow has decreased, and the degree of reflection of the light source and the subject may be decreased.

The performing of stereoscopic image processing may further include reflecting, as the dynamic information, an increase or decrease in the instantaneous variation amount of blood flow over time in a surface vector of the subject; and performing stereoscopic image processing by substituting, into a standard lighting model, the surface vector of the subject, in which the increase or decrease in the instantaneous variation amount of blood flow over time is reflected.

The method may further include receiving a user operation signal and adjusting a light source effect.

A method of processing ultrasound images, according to another embodiment, includes acquiring blood flow data from a subject including a region of interest; calculating static information of blood flow, which is independent of time changes, and dynamic information of blood flow, which changes over time, by analyzing the blood flow data; performing stereoscopic image processing such that a static flow of blood flow using the calculated static information and a dynamic flow of blood flow using the calculated dynamic information are expressed; and outputting a stereoscopically processed blood flow image, wherein the dynamic information includes a distribution of instantaneous variation amounts of blood flow.

The method may further include expressing, as the dynamic information, the distribution of instantaneous variation amounts of blood flow, and expressing a change in the distribution over time.

The performing of stereoscopic image processing may include changing a position of a light source, as a light source effect, according to a positional change of blood flow over time, on the basis of the distribution of instantaneous variation amounts of blood flow which is the dynamic information, and when the distribution of instantaneous variation amounts changes, it may be determined that a position of blood flow has changed, and the position of the light source may be changed.

The performing of stereoscopic image processing may further include reflecting, as the dynamic information, a change in the distribution of instantaneous variation amounts of blood flow over time in a position vector of a light source; and performing stereoscopic image processing by substituting, into a standard lighting model, the position vector of the light source, in which the change in the distribution of instantaneous variation amounts of blood flow is reflected.

The method may further include receiving a user operation signal and adjusting a light source effect.

A method of processing ultrasound images, according to still another embodiment, includes acquiring blood flow data from a subject including a region of interest; calculating static information of blood flow, which is independent of time changes, and dynamic information of blood flow, which changes over time, by analyzing the blood flow data; performing stereoscopic image processing such that a static flow of blood flow using the calculated static information and a dynamic flow of blood flow using the calculated dynamic information are expressed; and outputting a stereoscopically processed blood flow image, wherein the dynamic information includes an instantaneous variation amount of blood flow and a distribution of instantaneous variation amounts of blood flow.

The method may further include expressing the dynamic information, wherein the expressing of dynamic information may comprise, for blood flow data at a predetermined time, when the instantaneous variation amount of blood flow as the dynamic information has a positive (+) value, expressing the instantaneous variation amount as increasing, and, when the instantaneous variation amount of blood flow as the dynamic information has a negative (-) value, expressing the instantaneous variation amount as decreasing.

The method may further include expressing, as the dynamic information, the distribution of instantaneous variation amounts of blood flow, and expressing a change in the distribution of instantaneous variation amounts over time.

The performing of stereoscopic image processing may include increasing or decreasing an intensity of a light source, as a light source effect, according to a change in blood flow intensity over time, on the basis of the instantaneous variation amount of blood flow which is the dynamic information, when the instantaneous variation amount increases in a positive (+) direction, it may be determined that the blood flow intensity has increased and the intensity of the light source may be increased, and when the instantaneous variation amount increases in a negative (-) direction, it may be determined that the blood flow intensity has decreased, and the intensity of the light source may be decreased.

The performing of stereoscopic image processing may include changing a position of a light source, as a light source effect, according to a positional change of blood flow over time, on the basis of the distribution of instantaneous variation amounts of blood flow which is the dynamic information, and when the distribution of instantaneous variation amounts changes, it may be determined that a position of blood flow has changed, and the position of the light source may be changed.

The performing of stereoscopic image processing may include adjusting a degree of reflection of a light source and the subject, as a light source effect, according to a change in an amount of blood flow over time, on the basis of the instantaneous variation amount of blood flow which is the dynamic information, when the instantaneous variation amount increases in a positive (+) direction, it may be determined that the amount of blood flow has increased and the degree of reflection of the light source and the subject may be increased, and when the instantaneous variation amount increases in a negative (-) direction, it may be determined that the amount of blood flow has decreased, and the degree of reflection of the light source and the subject may be decreased.

The performing of stereoscopic image processing may further include reflecting, as the dynamic information, an increase or decrease in the instantaneous variation amount of blood flow over time in a surface vector of the subject; reflecting, as the dynamic information, a change in the distribution of instantaneous variation amounts of blood flow over time in a position vector of a light source; and performing stereoscopic image processing by substituting, into a standard lighting model, the surface vector of the subject, in which the increase or decrease in the instantaneous variation amount of blood flow is reflected, and the position vector of the light source, in which the change in the distribution of instantaneous variation amounts of blood flow is reflected.

The method may further include receiving a user operation signal and adjusting a light source effect.

An apparatus for processing ultrasound image, according to yet another embodiment, includes a data acquisition unit configured to acquire blood flow data from a subject including a region of interest; an information processing unit configured to calculate static information of blood flow, which is independent of time changes, and dynamic information of blood flow, which changes over time, by analyzing the blood flow data; an image processing unit configured to perform stereoscopic image processing such that a static flow and dynamic flow of blood flow are expressed in a blood flow image generated from the blood flow data, using the calculated static and dynamic information; and an output unit configured to output a stereoscopically processed blood flow image, wherein the dynamic information includes at least one of an instantaneous variation amount of blood flow or a distribution of instantaneous variation amounts of blood flow.

### [Advantages and Effects]

According to an apparatus and method for processing ultrasound images in accordance with an embodiment, by performing stereoscopic image processing on a planar blood flow image obtained through ultrasound while reflecting a lighting effect in which the dynamic flow of blood flow is represented, the blood flow image can be dynamically and stereoscopically expressed.

An apparatus and method for processing ultrasound images according to an embodiment provide stereoscopic image processing in which the static flow of blood flow on the basis of static information of blood flow data, which is independent of time changes, and the dynamic flow of blood flow on the basis of dynamic information of the blood flow data, which changes over time, are expressed, thereby dynamically and stereoscopically expressing a blood flow image.

Furthermore, an apparatus and method for processing ultrasound images according to an embodiment visually provide dynamic information for a blood flow image, so that a user can readily identify the structural form of the blood flow.

### [Description of Drawings]

FIG. 1 is a diagram illustrating the configuration of an apparatus for processing ultrasound images according to an embodiment of the present invention.
FIG. 2 is a flowchart illustrating a method of processing ultrasound images according to an embodiment of the present invention.
FIG. 3 is a diagram illustrating data processed according to the flow of the method shown in FIG. 2, according to an embodiment of the present invention.
FIG. 4 is a diagram illustrating an example of calculating dynamic information according to an embodiment of the present invention.
FIG. 5 illustrates an instantaneous variation amount according to an embodiment of the present invention.
FIG. 6 is a diagram illustrating a distribution of instantaneous variation amounts according to an embodiment of the present invention.
FIG. 7 is a diagram illustrating an example of dynamic stereoscopic image processing through an increase or decrease in the intensity of a light source according to an embodiment of the present invention.
FIG. 8 is a diagram illustrating an example of dynamic stereoscopic image processing through changing a position of a light source according to an embodiment of the present invention.
FIG. 9 is a diagram illustrating an example of dynamic stereoscopic image processing through an increase or decrease in the degree of reflection of a light source and a subject according to an embodiment of the present invention.
FIG. 10 is a diagram illustrating a blood flow image in which dynamic stereoscopic image processing of blood flow is performed according to an embodiment of the present invention.
FIG. 11 is a diagram illustrating a dynamic stereoscopic image processing process according to an embodiment of the present invention.
FIG. 12 is a diagram illustrating a standard lighting model according to an embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be constructed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present, and these terms may be varied according to the intent or custom of a user or an operator. Therefore, the definitions of the terms used herein should follow contexts disclosed herein.

Each block of the accompanying block diagram and combinations of each step of the accompanying flowchart may also be performed by computer program instructions (execution engines), which may be stored in a general-purpose computer, special purpose computer, or a processor of other programmable data processing device. In addition, the instructions that are executed through the computer or the processor of the other programmable data processing device generate means for performing the functions described in each block of the block diagram or in each step of the flowchart.

These computer program instructions may also be stored in a computer usable or computer readable memory capable of supporting a computer or the processor of other programmable data processing device to implement the function in a particular manner. Therefore the instructions stored in the computer usable or computer readable memory are possible to produce a manufacturing item containing instruction means for performing the function described in each block of the block diagram or in each step of the flowchart.

In addition, because the computer program instructions may also be loaded onto the computer or the other programmable data processing device, a series of operating steps is performed on a computer or other programmable data processing device, thereby creating a process that is executed by the computer. Here, the instructions executing the computer or the other programmable data processing device are also possible to provide steps to perform the functions described in each block of the block diagram and at each step of the flowchart.

In addition, each block or each step may represent a portion of a module, segment, or code including one or more executable instructions for executing one or more specified logical functions, and in some alternative embodiments, it should be noted that functions described in the blocks or the steps may occur in a different order. For example, two successive blocks or steps may actually be performed substantially concurrently, and it is also possible that the blocks or steps are performed in the reverse order of their corresponding functions, as needed.

Hereinafter, embodiments of the present invention will be described in detail with reference to the accompanying drawings. However, the present invention may be realized in various forms, and the scope of the present invention is not limited to such embodiments. The embodiments of the present invention are provided to aid those skilled in the art in the explanation and the understanding of the present invention.

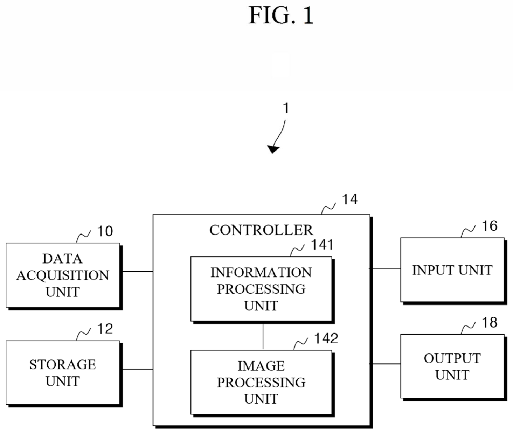

FIG. 1 is a diagram illustrating the configuration of an apparatus for processing ultrasound images according to an embodiment of the present invention.

An apparatus 1 for processing ultrasound images acquires ultrasound data of blood flow (hereinafter referred to as "blood flow data") from a subject including a region of interest, and generates and provides a blood flow image obtained by visualizing the acquired blood flow data. For example, the apparatus 1 may generate and provide microvascular imaging (MVI), which visualizes blood flow signals of microvessels.

The apparatus 1 for processing ultrasound images has non-invasive and nondestructive characteristics, and is therefore widely used in the medical field for obtaining internal information of a subject. Without the need to directly incise the subject for observation through surgical procedures, the apparatus 1 for processing ultrasound images can provide physicians with high-resolution images of the inside of a subject in real time, and thus plays a highly important role in the medical field.

The apparatus 1 for processing ultrasound images provides B-mode (brightness mode) images that display reflection coefficients of ultrasonic echo signals reflected from the subject as two-dimensional images, D-mode (Doppler mode) images that display, as Doppler spectra, the velocities of a moving object using the Doppler effect, C-mode (color Doppler mode) images that display, in color, the velocities of a moving object using the Doppler effect;
and elasticity-mode images that display differences in response before and after compression of the subject. In particular, the apparatus 1 for processing ultrasound images may transmit an ultrasonic signal to a subject including an object of interest, receive ultrasonic echo signals reflected from the subject, form a Doppler signal, and generate a C-mode image, that is, a color Doppler mode image, based on the formed Doppler signal.

According to one embodiment of the present invention, the apparatus 1 for processing ultrasound images may dynamically and stereoscopically express a blood flow image by performing static stereoscopic image processing in which a static flow of blood flow is expressed in a planar blood flow image obtained through ultrasound, together with dynamic stereoscopic image processing in which a dynamic flow of blood flow is expressed. Hereinafter, the configuration of the apparatus 1 for processing ultrasound images will be described.

Referring to FIG. 1, the apparatus 1 for processing ultrasound images includes a data acquisition unit 10, a storage unit 12, a controller 14, an input unit 16, and an output unit 18. The controller 14 may include an information processing unit 141 and an image processing unit 142.

The data acquisition unit 10 transmits ultrasonic signals to a subject including a region of interest through an ultrasound probe, and receives ultrasonic echo signals reflected from the subject to acquire blood flow data. The blood flow data may be ensemble data acquired over a predetermined period of time.

The ultrasound probe includes a plurality of transducer elements that operate to mutually convert electrical signals and ultrasonic signals. The ultrasound probe transmits ultrasonic signals to the subject and receives ultrasonic echo signals reflected from the subject to form a received signal. The received signal is an analog signal. The ultrasound probe may include, for example, a convex probe, a linear probe, and the like.

The data acquisition unit 10 may perform: a process of receiving ultrasonic echo signals reflected from the subject through the ultrasound probe and converting the ultrasonic echo signals into electrical received signals; a process of time-delaying the received signals and summing the time-delayed received signals to output a reception focused beam; and a process of generating blood flow data corresponding to a blood flow image by performing image signal processing based on the output reception focused beam.

According to an embodiment, the controller 14 performs static stereoscopic image processing, which expresses a static flow of blood flow in the blood flow image on the basis of static information of the blood flow data, together with dynamic stereoscopic image processing, which expresses a dynamic flow of blood flow on the basis of dynamic information of the blood flow data that changes over time. Accordingly, the blood flow image can be expressed dynamically and stereoscopically.

The static information is a value of a component among the blood flow data components that is independent of time changes, and is either a component value of the blood flow data at a predetermined time or a value obtained by averaging blood flow data component values over a certain period of time. For example, the static information may be information obtained by averaging velocity, power, and variance components of the blood flow data over a certain period of time.

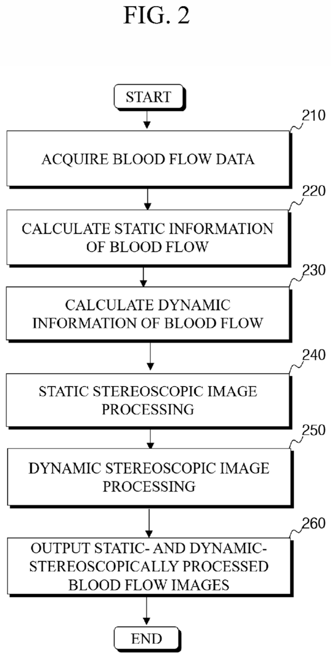

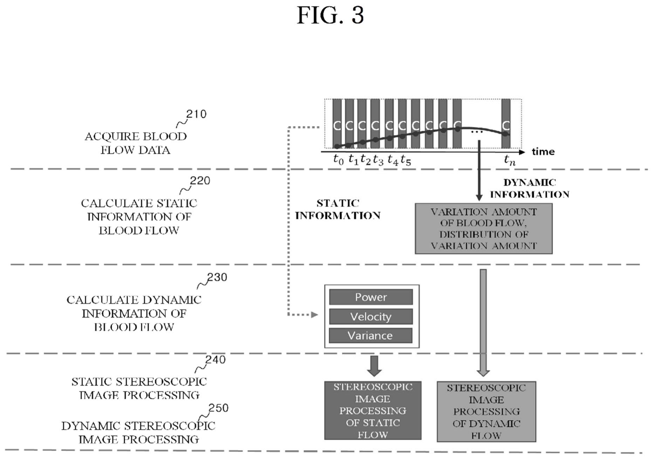

The dynamic information is a value of a component among the blood flow data components that varies with time. For example, the dynamic information may include an instantaneous variation amount of blood flow and a distribution of instantaneous variation amounts of blood flow. The dynamic information may be calculated, for blood flow data acquired over a certain period of time, based on a difference between previous blood flow data and current blood flow data. Examples of the dynamic information will be described below with reference to FIGS. 2 and 3.

The controller 14 performs image processing on the blood flow data acquired by the data acquisition unit 10 to generate a stereoscopic and dynamic blood flow image, and transfers the generated blood flow image to the output unit 18. For example, the blood flow image may be a color Doppler mode image.

According to an embodiment, the controller 14 may include an information processing unit 141 and an image processing unit 142.

The information processing unit 141 analyzes blood flow data to calculate dynamic information of blood flow that changes over time and static information of blood flow that is independent of time changes.

The image processing unit 142 performs static stereoscopic image processing such that a static flow of blood flow is expressed in the blood flow image generated from the blood flow data, using the static information calculated by the information processing unit 141. In addition, the image processing unit 142 performs dynamic stereoscopic image processing such that a dynamic flow of blood flow is expressed in the blood flow image generated from the blood flow data, using the dynamic information calculated by the information processing unit 141. The static stereoscopic image processing is a representation in which a static flow of blood flow is three-dimensionally expressed in a two-dimensional blood flow image. The dynamic stereoscopic image processing is a representation in which blood flow is three-dimensionally expressed in a two-dimensional blood flow image.

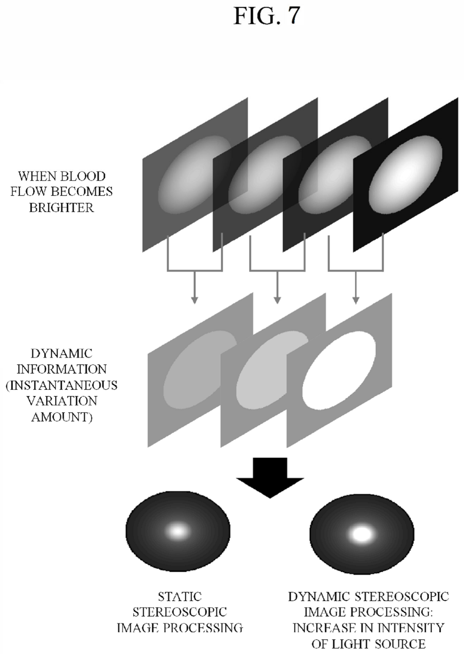

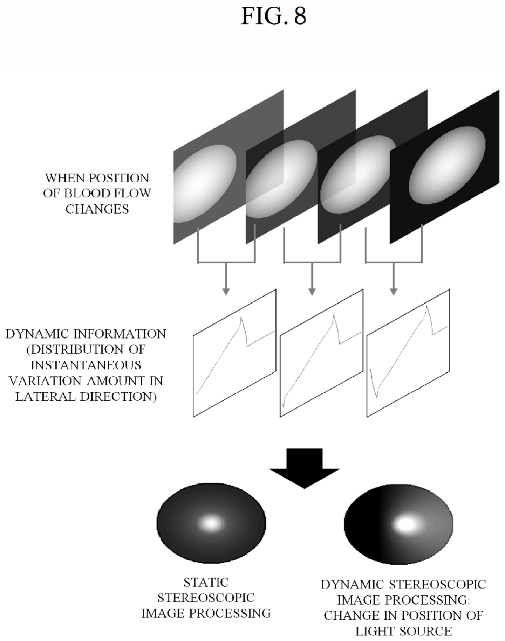

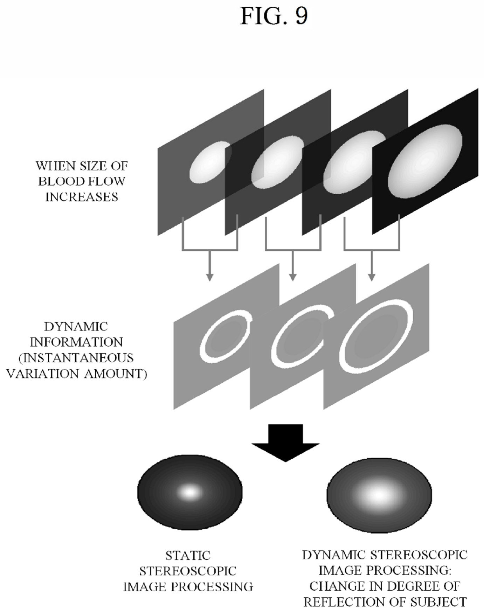

The dynamic stereoscopic image processing may be achieved by applying a light source effect to the blood flow image based on the dynamic information. The light source effect includes, for example, an increase or decrease in intensity of a light source, a change in position of the light source, and an increase or decrease in the degree of reflection of the light source and a subject. The light source may be, for example, illumination. The subject may include blood flow. By reflecting such characteristics of the light source using the dynamic information, the image processing unit 142 may express blood flow in a stereoscopic and dynamic manner. Details of dynamic stereoscopic image processing through increasing and decreasing the intensity of the light source will be described below with reference to FIG. 7, details of dynamic stereoscopic image processing through changing the position of the light source will be described below with reference to FIG. 8, and details of dynamic stereoscopic image processing through increasing and decreasing the degree of reflection of the light source and the subject will be described below with reference to FIG. 9.

The input unit 16 provides an interface for receiving input information from a user. In an embodiment, the interface allows the user to make a selection with respect to the blood flow image of the subject and size and position information of a region of interest (that is, a color box) that is set on the blood flow image. A user input unit 130 may include a control panel, a mouse, a keyboard, and the like.

The output unit 18 outputs, to a screen for the user, the blood flow image formed by the controller 14. The blood flow image is an image reconstructed according to estimation of blood flow data by the controller 14.

The storage unit 12 stores an operation process of the apparatus 1 for processing ultrasound images according to an embodiment of the present invention, and may be implemented by one or more of a conventional hard disk, random access memory (RAM), or read-only memory (ROM).

FIG. 2 is a flowchart illustrating a method of processing ultrasound images according to an embodiment of the present invention, and FIG. 3 is a diagram illustrating data processed according to the flow of the method shown in FIG. 2 according to an embodiment of the present invention.

Referring to FIGS. 1 to 3, the apparatus 1 for processing ultrasound images acquires blood flow data from a subject including a region of interest (210). The blood flow data may be ensemble data acquired over a predetermined period of time, for example, from time t₀ to time tₙ.

Subsequently, the apparatus 1 for processing ultrasound images analyzes the blood flow data to calculate static information of blood flow that is independent of time changes and dynamic information of blood flow that changes over time (220). At this time, the calculation of the static information and the calculation of the dynamic information may be performed in any order.

The static information is a value of a component among the blood flow data components that is independent of time changes, and is either a component value of the blood flow data at a predetermined time or a value obtained by averaging blood flow data component values over a certain period of time. For example, the static information may be information obtained by averaging velocity, power, and variance components of the blood flow data over a certain period of time.

The dynamic information is a value of a component among the blood flow data components that varies with time. The dynamic information may include an instantaneous variation amount of blood flow and a distribution of instantaneous variation amounts of blood flow.

The "instantaneous variation amount of blood flow" refers to an instantaneous variation amount between respective blood flow data. The apparatus 1 for processing ultrasound images may calculate the instantaneous variation amount of blood flow based on a difference between previous blood flow data and current blood flow data. For example, among N pieces of blood flow data, an instantaneous variation amount of the (N-1)ₜₕ blood flow data may be {Nₜₕ blood flow data (fₙ) - (N-1)ₜₕ blood flow data (fₙ₋₁)}. The apparatus 1 for processing ultrasound images may obtain the instantaneous variation amount of blood flow by calculating a magnitude value of the instantaneous variation amount at each time or an average of the instantaneous variation amounts over the entire time.

The instantaneous variation amount of blood flow enables identification of blood-flow intensity, an amount of blood flow, and the like. The "instantaneous variation amount of blood flow" may be expressed as a positive (+) or negative (-) value. For blood flow data at a predetermined time, when the instantaneous variation amount of blood flow has a positive (+) value, the instantaneous variation amount may be expressed as increasing. In contrast, when the instantaneous variation amount of blood flow has a negative (-) value, the instantaneous variation amount may be expressed as decreasing. When the blood-flow intensity increases over time, the instantaneous variation amount increases in a positive (+) direction. In contrast, when the blood-flow intensity decreases, the instantaneous variation amount increases in a negative (-) direction. When the amount of blood flow increases over time, the instantaneous variation amount increases in the positive (+) direction. In contrast, when the amount of blood flow decreases, the instantaneous variation amount increases in the negative (-) direction.

The "distribution of instantaneous variation amounts of blood flow" refers to a degree of distribution of instantaneous variation amounts of blood flow with respect to a predetermined reference direction. The reference direction may be, for example, a lateral direction or an axial direction. The distribution may include, for example, variance, deviation, and standard deviation. The distribution of instantaneous variation amounts of blood flow enables identification of positional changes of blood flow over time. For example, when the position of blood flow changes over time, the distribution of instantaneous variation amounts changes.

The apparatus 1 for processing ultrasound images may express the calculated dynamic information. For example, for blood flow data at a predetermined time, when the instantaneous variation amount of blood flow as the dynamic information has a positive (+) value, the apparatus 1 for processing ultrasound images may express the instantaneous variation amount as increasing. In contrast, when the instantaneous variation amount of blood flow has a negative (-) value, the instantaneous variation amount may be expressed as decreasing.

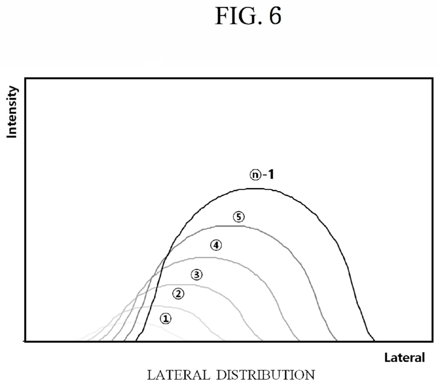

In another example, the apparatus 1 for processing ultrasound images may express, as the dynamic information, a distribution of instantaneous variation amounts of blood flow. For example, a change in the distribution with time may be expressed. An embodiment relating thereto will be described below with reference to FIG. 6.

Subsequently, the apparatus 1 for processing ultrasound images performs stereoscopic image processing such that a static flow of blood flow using the calculated static information and a dynamic flow of blood flow using the calculated dynamic information are expressed (230).

In the stereoscopic image processing step 230, the apparatus 1 for processing ultrasound images may increase or decrease an intensity of a light source, as a light source effect, according to a change in blood flow intensity over time, on the basis of the instantaneous variation amount of blood flow which is the dynamic information. For example, when the instantaneous variation amount increases in the positive (+) direction in predetermined blood flow data, it is determined that the blood-flow intensity has increased, and the intensity of the light source is increased. In contrast, when the instantaneous variation amount increases in the negative (-) direction, it is determined that the blood-flow intensity has decreased, and the intensity of the light source is decreased. An embodiment relating thereto will be described below with reference to FIG. 7.

In the stereoscopic image processing step 230, the apparatus 1 for processing ultrasound images may change a position of the light source, as a light source effect, according to a positional change of blood flow over time, on the basis of a distribution of instantaneous variation amounts of blood flow which is the dynamic information. For example, when the distribution of instantaneous variation amounts changes, it is determined that the position of the blood flow has changed, and the position of the light source is changed. An embodiment relating thereto will be described below with reference to FIG. 8.

In the stereoscopic image processing step 230, the apparatus 1 for processing ultrasound images may adjust the degree of reflection of the light source and the subject, as a light source effect, according to a change in an amount of blood flow over time, on the basis of the instantaneous variation amount of blood flow which is the dynamic information. For example, when the instantaneous variation amount increases in the positive (+) direction in predetermined blood flow data, it is determined that the amount of blood flow has increased, and the degree of reflection of the light source and the subject is increased. In contrast, when the instantaneous variation amount increases in the negative (-) direction, it is determined that the amount of blood flow has decreased, and the degree of reflection of the light source and the subject is decreased. An embodiment relating thereto will be described below with reference to FIG. 9.

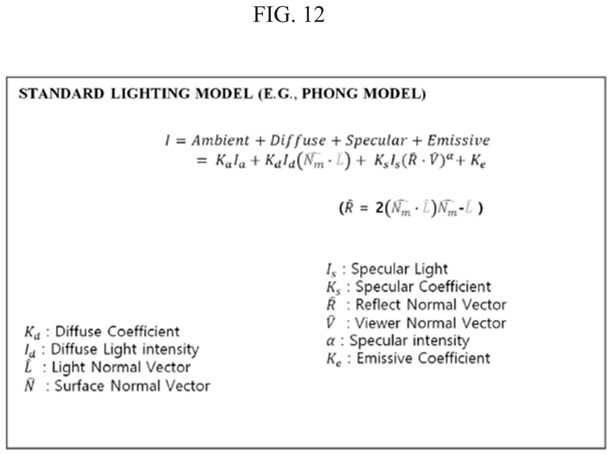

In the stereoscopic image processing step 230, the apparatus 1 for processing ultrasound images may reflect, as the dynamic information, an increase and a decrease in an instantaneous variation amount of blood flow over time in a surface vector of the subject. In addition, as the dynamic information, a change in a distribution of instantaneous variation amounts of blood flow over time may be reflected in a position vector of the light source. Subsequently, dynamic stereoscopic image processing may be performed by substituting the surface vector of the subject, in which the increase or decrease in the instantaneous variation amount of blood flow is reflected, and the position vector of the light source, in which the change in the distribution of instantaneous variation amounts of blood flow is reflected, into a standard lighting model. The surface vector of the subject, the position vector of the light source, and the standard lighting model will be described below with reference to FIG. 12.

Subsequently, the apparatus 1 for processing ultrasound images outputs the stereoscopically processed blood flow image (240).

Thereafter, the apparatus 1 may receive a user operation signal and adjust the light source effect. For example, after the user checks the blood flow image in which the light source effect is reflected by the apparatus 1 for processing ultrasound images, the user may increase or decrease an intensity of the light source, increase or decrease the degree of reflection of the light source and the subject, or change a position of the light source.

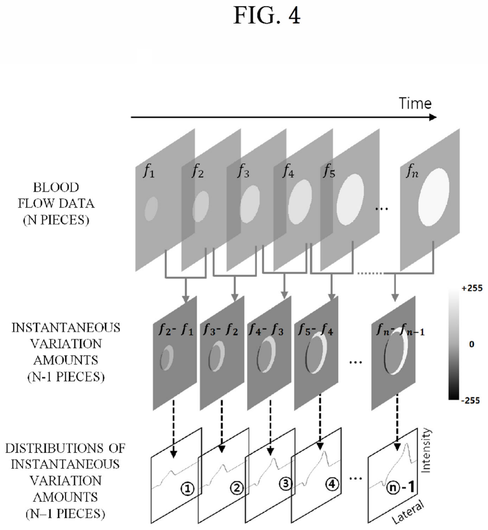

FIG. 4 is a diagram illustrating an example of calculating dynamic information according to an embodiment of the present invention.

Referring to FIGS. 1 and 4, the apparatus 1 for processing ultrasound images calculates dynamic information from blood flow data for a region of interest, for example, from ensemble data acquired over a predetermined period (from time t₀ to tₙ).

The "instantaneous variation amount of blood flow," which is one type of dynamic information, refers to an instantaneous variation amount between respective blood flow data. The apparatus 1 for processing ultrasound images may calculate the instantaneous variation amount of blood flow based on a difference between previous blood flow data and current blood flow data. For example, as illustrated in FIG. 4, among N pieces of blood flow data (f₁, f₂, ..., fₙ₋₁, fₙ), an instantaneous variation amount of the (N-1)ₜₕ blood flow data is {Nₜₕ blood flow data (fₙ) - (N-1)ₜₕ blood flow data (fₙ₋₁)}.

The "distribution of instantaneous variation amounts of blood flow," which is another type of dynamic information, refers to a degree of distribution of instantaneous variation amounts of blood flow with respect to a predetermined reference direction. The reference direction may be, for example, a lateral direction or an axial direction. The distribution may include, for example, variance, deviation, and standard deviation. FIG. 4 illustrates a lateral-direction distribution of instantaneous variation amounts of blood flow.

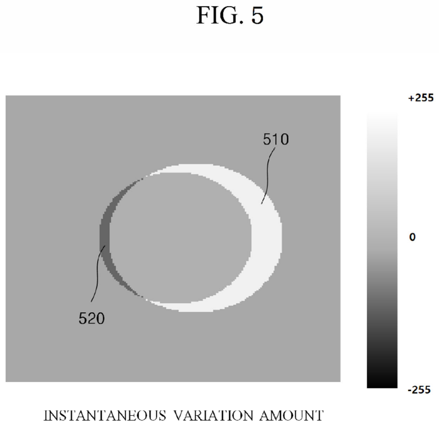

FIG. 5 illustrates an instantaneous variation amount according to an embodiment of the present invention.

Referring to FIGS. 1, 4, and 5, the apparatus 1 for processing ultrasound images may express the "instantaneous variation amount of blood flow" as a positive (+) or negative (-) value. For example, among N pieces of blood flow data f₁, f₂, ..., fₙ₋₁, and fₙ in FIG. 4, when, for blood flow data at a predetermined time, the "instantaneous variation amount of blood flow" has a positive (+) value, the instantaneous variation amount may be expressed as increasing. In contrast, when the "instantaneous variation amount of blood flow" has a negative (-) value, the instantaneous variation amount may be expressed as decreasing. The apparatus 1 for processing ultrasound images may define an image region 510 in which the instantaneous variation amount has a positive (+) value as a region in which the instantaneous variation amount of blood flow increases, and may define an image region 520 in which the instantaneous variation amount has a negative (-) value as a region in which the instantaneous variation amount of blood flow decreases.

FIG. 6 is a diagram illustrating a distribution of instantaneous variation amounts according to an embodiment of the present invention.

Referring to FIGS. 1, 4 and 6, the apparatus 1 for processing ultrasound images may display a distribution of instantaneous variation amounts of blood flow over time. For example, as illustrated in FIG. 6, a distribution in the lateral direction of instantaneous variation amounts of blood flow may be displayed. As the time changes (① to ⓝ-1) in FIG. 4, the distribution of instantaneous variation amounts changes from left to right as illustrated in FIG. 6, and the intensity increases.

FIG. 7 is a diagram illustrating an example of dynamic stereoscopic image processing through an increase or decrease in the intensity of a light source according to an embodiment of the present invention.

Referring to FIGS. 1 and 7, the apparatus 1 for processing ultrasound images may dynamic-stereoscopically process a blood flow image by increasing or decreasing an intensity of a light source on the basis of the instantaneous variation amount of blood flow over time.

For example, as illustrated in FIG. 7, the apparatus 1 for processing ultrasound images may dynamic-stereoscopically process a blood flow image by increasing or decreasing the intensity of a light source according to a change in blood-flow intensity over time, on the basis of the instantaneous variation amount of blood flow. For example, when the instantaneous variation amount increases in a positive (+) direction in predetermined blood flow data, it is determined that the blood-flow intensity has increased, and the intensity of the light source is increased. In contrast, when the instantaneous variation amount increases in the negative (-) direction in the predetermined blood flow data, it is determined that the blood-flow intensity has decreased, and the intensity of the light source is decreased. FIG. 7 illustrates an example in which an illumination intensity is increased as the blood-flow intensity increases.

FIG. 8 is a diagram illustrating an example of dynamic stereoscopic image processing through changing a position of a light source according to an embodiment of the present invention.

Referring to FIGS. 1 and 8, the apparatus 1 for processing ultrasound images may change the position of the light source according to a positional change of blood flow over time, on the basis of a distribution of instantaneous variation amounts of blood flow. For example, when the distribution of instantaneous variation amounts changes, the apparatus 1 for processing ultrasound images determines that the position of the blood flow has changed, and changes the position of the light source. FIG. 8 illustrates an example in which, when the distribution of instantaneous variation amounts changes from left to right, the position of the light source is changed from left to right.

FIG. 9 is a diagram illustrating an example of dynamic stereoscopic image processing through an increase or decrease in the degree of reflection of a light source and a subject according to an embodiment of the present invention.

Referring to FIGS. 1 and 9, the apparatus 1 for processing ultrasound images adjusts a degree of reflection of the light source and the subject according to a change in an amount of blood flow over time, on the basis of an instantaneous variation amount of blood flow. For example, when the instantaneous variation amount increases in a positive (+) direction, the apparatus 1 for processing ultrasound images determines that the amount of blood flow has increased, and increases the degree of reflection of the light source and the subject. In contrast, when the instantaneous variation amount increases in a negative (-) direction, the apparatus 1 for processing ultrasound images determines that the amount of blood flow has decreased, and decreases the degree of reflection of the light source and the subject. The degree of reflection of the light source and the subject can be adjusted by controlling a reflection angle. FIG. 9 illustrates an example in which, when the amount of blood flow increases, the degree of reflection of the light source and the subject is greatly adjusted.

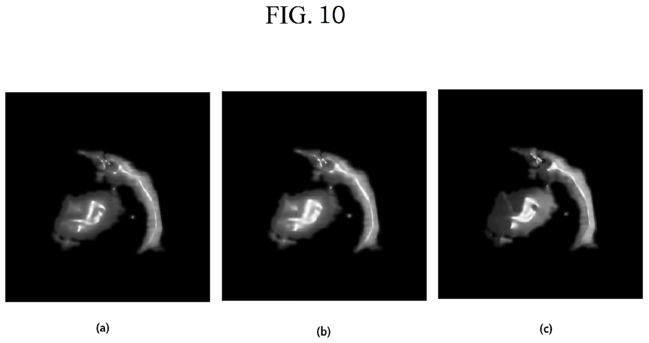

FIG. 10 is a diagram illustrating a blood flow image in which dynamic stereoscopic image processing of blood flow is performed according to an embodiment of the present invention.

Referring to FIGS. 1 and 10, the apparatus 1 for processing ultrasound images may generate a stereoscopic and dynamic blood flow image by applying dynamic stereoscopic image processing to a blood flow image such that a dynamic flow of blood flow is expressed therein using dynamic information.

For example, when a blood-flow intensity (strength) increases, the apparatus 1 for processing ultrasound images increases the intensity of a light source as an instantaneous variation amount of blood flow increases in a positive (+) direction. In contrast, when the blood-flow intensity (strength) decreases, the apparatus 1 for processing ultrasound images decreases the intensity of the light source as the instantaneous variation amount of blood flow increases in a negative (-) direction.

In another example, when a position of blood flow changes, the apparatus 1 for processing ultrasound images changes a position of the light source as a distribution of instantaneous variation amounts of blood flow changes.

In still another example, when the amount of blood flow increases, the apparatus 1 for processing ultrasound images increases a degree of reflection of the subject and the light source as a range of the instantaneous variation amount of blood flow increases in the positive (+) direction. In contrast, when the amount of blood flow decreases, the apparatus 1 for processing ultrasound images decreases the degree of reflection of the subject and the light source as the range of the instantaneous variation amount of blood flow decreases in the negative (-) direction.

In FIG. 10, (a) is a blood flow image in which only static stereoscopic image processing is performed, (b) is a blood flow image to which dynamic stereoscopic image processing is applied on the assumption that the blood flow has increasing intensity and amount, and (c) is a blood flow image to which dynamic stereoscopic image processing is applied on the assumption that the blood flow is changing to the right. In the case of (b), the intensity of the light source is increased and the degree of reflection of the subject and the light source is increased for dynamic stereoscopic image processing. In the case of (c), the position of the light source is changed for dynamic stereoscopic image processing. Compared with (a), in which only static stereoscopic image processing is performed, when dynamic stereoscopic image processing is additionally performed as in (b) and (c), a dynamic and stereoscopic effect may be obtained. For example, even when a blood vessel has a small diameter, a dynamic, stereoscopically processed blood flow image may be generated by applying a strong lighting effect to a blood vessel having a large amount of blood flow.

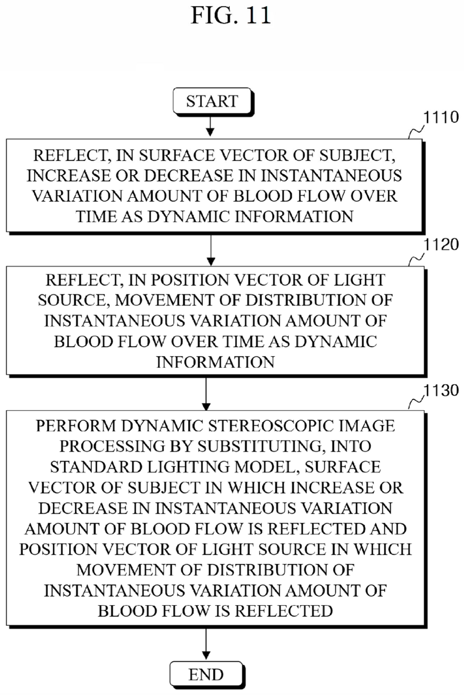

FIG. 11 is a diagram illustrating a dynamic stereoscopic image processing process according to an embodiment of the present invention, and FIG. 12 is a diagram illustrating a standard lighting model according to an embodiment of the present invention.

Referring to FIGS. 1, 2, 11, and 12, in the stereoscopic image processing step 230 of FIG. 2, the apparatus 1 for processing ultrasound images reflects, in a surface vector *N̂ₘ* of a subject, an increase or decrease in an instantaneous variation amount of blood flow over time which is dynamic information (1110).

Next, the apparatus 1 for processing ultrasound images reflects, in a position vector (Lₓ, L_{y}, L_{z}) of the light source, a change in a distribution of instantaneous variation amounts of blood flow over time which is the dynamic information (1120).

Thereafter, the apparatus 1 for processing ultrasound images performs dynamic stereoscopic image processing by substituting, into a standard lighting model, the surface vector *N̂ₘ* of the subject in which the increase or decrease in the instantaneous variation amount of blood flow is reflected, and the position vector *L̂* (Lₓ, L_{y}, L_{z}) of the light source in which the change in the distribution of instantaneous variation amounts of blood flow is reflected (1130).

The apparatus 1 for processing ultrasound images may adjust, based on the instantaneous variation amount, the intensity of the light source and the degree of reflection of the light source and the subject by reflecting, in the surface vector *N̂ₘ* , a variation value of the instantaneous variation amount, which is the dynamic information. In addition, the apparatus 1 for processing ultrasound images may adjust a change in the position of the light source based on the distribution of the instantaneous variation amounts by reflecting, in the position vector *L̂* (Lₓ, L_{y}, L_{z}) of the light source, a change in the distribution of the instantaneous variation amounts which is the dynamic information. Nₓ is a gradient in a scanline direction of the instantaneous variation-amount information of blood flow, N_{y} is a gradient in a sample direction of the instantaneous variation-amount information of blood flow, and N_{z} is the instantaneous variation amount of blood flow.

The apparatus 1 for processing ultrasound images performs dynamic stereoscopic image processing by substituting the surface vector *N̂ₘ* of the subject, in which the dynamic information is reflected, and the position vector *L̂* (Lₓ, L_{y}, L_{z}) of the light source, in which the dynamic information is reflected, into a standard lighting model. The standard lighting model is illustrated in FIG. 12.

Heretofore, the present invention has been described by focusing on the exemplary embodiments. It can be understood by those skilled in the art to which the present invention pertains that the present invention can be implemented in modified forms without departing from the essential feature of the present invention. Therefore, the disclosed embodiments should be considered as illustrative rather than determinative. The scope of the present invention is defined by the appended claims rather than by the foregoing description, and all differences within the scope of equivalents thereof should be constructed as being included in the present invention.

## Claims

1. A method of processing ultrasound images, performed by an apparatus for processing ultrasound image, the method comprising:
acquiring ultrasound data of blood flow (hereinafter referred to as "blood flow data") from a subject including a region of interest;
calculating static information of blood flow, which is independent of time changes, and dynamic information of blood flow, which changes over time, by analyzing the blood flow data;
performing stereoscopic image processing such that a static flow of blood flow using the calculated static information and a dynamic flow of blood flow using the calculated dynamic information are expressed; and
outputting a stereoscopically processed blood flow image,
wherein the dynamic information includes an instantaneous variation amount of blood flow.

2. The method of claim 1, further comprising expressing the dynamic information,
wherein the expressing of the dynamic information comprises, for blood flow data at a predetermined time, when the instantaneous variation amount of blood flow as the dynamic information has a positive (+) value, expressing the instantaneous variation amount as increasing, and when the instantaneous variation amount of blood flow as the dynamic information has a negative (-) value, expressing the instantaneous variation amount as decreasing.

3. The method of claim 1, wherein the performing of stereoscopic image processing comprises increasing or decreasing an intensity of a light source, as a light source effect, according to a change in blood flow intensity over time, on the basis of the instantaneous variation amount of blood flow which is the dynamic information,
when the instantaneous variation amount increases in a positive (+) direction, it is determined that the blood flow intensity has increased and the intensity of the light source is increased, and
when the instantaneous variation amount increases in a negative (-) direction, it is determined that the blood flow intensity has decreased, and the intensity of the light source is decreased.

4. The method of claim 1, wherein the performing of stereoscopic image processing comprises adjusting a degree of reflection of a light source and the subject, as a light source effect, according to a change in an amount of blood flow over time, on the basis of the instantaneous variation amount of blood flow which is the dynamic information,
when the instantaneous variation amount increases in a positive (+) direction, it is determined that the amount of blood flow has increased and the degree of reflection of the light source and the subject is increased, and
when the instantaneous variation amount increases in a negative (-) direction, it is determined that the amount of blood flow has decreased, and the degree of reflection of the light source and the subject is decreased.

5. The method of claim 1, wherein the performing of stereoscopic image processing comprises:
reflecting, as the dynamic information, an increase or decrease in the instantaneous variation amount of blood flow over time in a surface vector of the subject; and
performing dynamic stereoscopic image processing by substituting, into a standard lighting model, the surface vector of the subject, in which the increase or decrease in the instantaneous variation amount of blood flow over time is reflected.

6. The method of claim 1, further comprising receiving a user operation signal and adjusting a light source effect.

7. A method of processing ultrasound images, performed by an apparatus for processing ultrasound image, the method comprising:
acquiring blood flow data from a subject including a region of interest;
calculating static information of blood flow, which is independent of time changes, and dynamic information of blood flow, which changes over time, by analyzing the blood flow data;
performing stereoscopic image processing such that a static flow of blood flow using the calculated static information and a dynamic flow of blood flow using the calculated dynamic information are expressed; and
outputting a stereoscopically processed blood flow image,
wherein the dynamic information includes a distribution of instantaneous variation amounts of blood flow.

8. The method of claim 7, further comprising expressing, as the dynamic information, the distribution of instantaneous variation amounts of blood flow, and expressing a change in the distribution of instantaneous variation amounts over time.

9. The method of claim 7, wherein the performing of stereoscopic image processing comprises changing a position of a light source, as a light source effect, according to a positional change of blood flow over time, on the basis of the distribution of instantaneous variation amounts of blood flow which is the dynamic information, and
when the distribution of instantaneous variation amounts changes, it is determined that a position of blood flow has changed, and the position of the light source is changed.

10. The method of claim 7, wherein the performing of stereoscopic image processing further comprises:
reflecting, as the dynamic information, a change in the distribution of instantaneous variation amounts of blood flow over time in a position vector of a light source; and
performing dynamic stereoscopic image processing by substituting, into a standard lighting model, the position vector of the light source, in which the change in the distribution of instantaneous variation amounts of blood flow is reflected.

11. The method of claim 7, further comprising receiving a user operation signal and adjusting a light source effect.

12. A method of processing ultrasound images, performed by an apparatus for processing ultrasound image, the method comprising:
acquiring blood flow data from a subject including a region of interest;
calculating static information of blood flow, which is independent of time changes, and dynamic information of blood flow, which changes over time, by analyzing the blood flow data;
performing stereoscopic image processing such that a static flow of blood flow using the calculated static information and a dynamic flow of blood flow using the calculated dynamic information are expressed; and
outputting a stereoscopically processed blood flow image,
wherein the dynamic information includes an instantaneous variation amount of blood flow and a distribution of instantaneous variation amounts of blood flow.

13. The method of claim 12, further comprising expressing the dynamic information,
wherein the expressing of the dynamic information comprises, for blood flow data at a predetermined time, when the instantaneous variation amount of blood flow as the dynamic information has a positive (+) value, expressing the instantaneous variation amount as increasing, and
when the instantaneous variation amount of blood flow as the dynamic information has a negative (-) value, expressing the instantaneous variation amount as decreasing.

14. The method of claim 12, further comprising expressing, as the dynamic information, a distribution of instantaneous variation amounts of blood flow, and expressing a change in the distribution of instantaneous variation amounts over time.

15. The method of claim 12, wherein the performing of stereoscopic image processing comprises increasing or decreasing an intensity of a light source, as a light source effect, according to a change in blood flow intensity over time, on the basis of an instantaneous variation amount of blood flow which is the dynamic information,
when the instantaneous variation amount increases in a positive (+) direction, it is determined that the blood flow intensity has increased and the intensity of the light source is increased, and
when the instantaneous variation amount increases in a negative (-) direction, it is determined that the blood flow intensity has decreased and the intensity of the light source is decreased.

16. The method of claim 12, wherein the performing of stereoscopic image processing comprises changing a position of a light source, as a light source effect, according to a positional change of blood flow over time, on the basis of the distribution of instantaneous variation amounts of blood flow which is the dynamic information, and
when the distribution of instantaneous variation amounts changes, it is determined that a position of blood flow has changed, and the position of the light source is changed.

17. The method of claim 12, wherein the performing of stereoscopic image processing comprises adjusting a degree of reflection of a light source and the subject, as a light source effect, according to a change in an amount of blood flow over time, on the basis of an instantaneous variation amount of blood flow which is the dynamic information,
when the instantaneous variation amount increases in a positive (+) direction, it is determined that the amount of blood flow has increased and the degree of reflection of the light source and the subject is increased, and
when the instantaneous variation amount increases in a negative (-) direction, it is determined that the amount of blood flow has decreased, and the degree of reflection of the light source and the subject is decreased.

18. The method of claim 12, wherein the performing of stereoscopic image processing further comprises:
reflecting, as the dynamic information, an increase or decrease in the instantaneous variation amount of blood flow over time in a surface vector of the subject;
reflecting, as the dynamic information, a change in the distribution of instantaneous variation amounts of blood flow over time in a position vector of a light source; and
performing dynamic stereoscopic image processing by substituting, into a standard lighting model, the surface vector of the subject, in which the increase or decrease in the instantaneous variation amount of blood flow is reflected, and the position vector of the light source, in which the change in the distribution of instantaneous variation amounts of blood flow is reflected.

19. The method of claim 12, further comprising receiving a user operation signal and adjusting a light source effect.

20. An apparatus for processing ultrasound images, comprising:
a data acquisition unit configured to acquire blood flow data from a subject including a region of interest;
an information processing unit configured to calculate static information of blood flow, which is independent of time changes, and dynamic information of blood flow, which changes over time, by analyzing the blood flow data;
an image processing unit configured to perform stereoscopic image processing such that a static flow and dynamic flow of blood flow are expressed in a blood flow image generated from the blood flow data, using the calculated static and dynamic information; and
an output unit configured to output a stereoscopically processed blood flow image,
wherein the dynamic information includes at least one of an instantaneous variation amount of blood flow or a distribution of instantaneous variation amounts of blood flow.
